# EUROPEAN PATENT SPECIFICATION

(11) **EP 2 464 703 B1**
(45) Date of publication and mention of the grant of the patent: **31.07.2019**
(21) Application number: 10808022.7
(22) Date of filing: 16.08.2010
(51) Int. Cl.: C10L 1/02, C09F 1/04

(54) **METHOD OF REFINING CRUDE TALL OIL**
VERFAHREN ZUM RAFFINIEREN VON ROHEM TALLÖL
PROCÉDÉ DE RAFFINAGE DE TALLOL BRUT

(30) Priority: 14.08.2009 FI 20095848
(43) Date of publication of application: 20.06.2012
(73) Proprietor: Forchem Oyj, 26101 Rauma (FI)
(72) Inventor: SAVIAINEN, Juhani, FI-26101 Rauma (FI); SAARENKO, Timo, FI-26101 Rauma (FI); RINTOLA, Mikko, FI-26101 Rauma (FI)
(74) Representative: Seppo Laine Oy
(86) International application number: PCT/FI2010/050635
(87) International publication number: WO 2011/018558

(56) References cited:
- EP-A1- 0 194 165
- US-A1- 2005 274 065
- US-A1- 2009 043 118

## Description

The present invention relates to the conversion of vegetable-based organic acids into corresponding lower alcohol esters.

According to such a method, a raw material which comprises organic acids is brought into contact with a lower alcohol, in the presence of an acidic catalyst, in order to esterify the acids.

It is already known that it is possible to produce, by esterification, biodiesel products from a vegetable-based acid composition, such as a crude fatty acid fraction of tall oil. Corresponding esters, such as methyl or ethyl esters, are formed from the acids, by using lower alcohols.

The known technology is described for instance in the publication E. Santacesaria & al (Ind Eng Chem Res 2005, 44, 9461-9472), which describes a solution where, in esterification, a 98 % conversion of the intitial materials is achieved with a retention time of 6 hours by using Celite CS ion-exchange resin as the catalyst.

The Swedish company Kiram Ab has applied for patent protection for a method, in which TOME ester (tall oil methyl ester) is produced, first in a reaction tank and then in a reactive distillation column, in which case, in the gaseous stage, the water generated is continuously removed with the aid of methanol (PCT published patent application WO/2007/050030). It should be noted that, in the process mentioned, the subject of esterification is crude tall oil (CTO) which, in the research carried out in the present invention, has been found to esterify poorly.

Another example of the known technology is described in an article in the magazine Journal of Applied Sciences & Environmental Management (J. Appl. Sci. Environ. Mgt. 2003, Vol. 7(1), 15-17), according to which article a 94 % conversion is achieved by the methylation of crude acid, the molar ratio between the reactants being 10 and the reaction time 1105 minutes, by using sulphuric acid as the catalyst.

A Japanese study (ISSN :0021-9592) suggests that it is possible to methylate the fatty acids without a catalyst, when the temperature is increased to 350 °C during a period of 240 seconds, in which case a 98 % conversion was achieved in the research.

US patent publication 5 349 075 describes a continuous working methanol esterification of fatty acids, carried out without a catalyst, in which case water was removed from the reaction with the aid of a gaseous stage, at a temperature of 200-260 °C. A 97 % conversion was achieved in three hours, the molar ratio between methanol and fatty acids being 6.

The Korean company SK Chemicals has applied for a patent on a solution in which fatty acids are continuously esterified at a temperature of 200-350 °C, also without a catalyst, the molar ratio of the MeOH/fatty acids being 20. In this respect, we refer to the international published patent application WO 2007/126166 A1.

PCT published patent application WO 2009/021248 describes how the percentage of free fatty acids in the raw material is decreased before the actual transesterification. Generation of soap is avoided because there are no longer any free fatty acids in the raw material of the feed, when the mixture enters into transesterification. The raw material is vegetable oil, which is primarily transesterified by using an alkali catalyst. The mixer device is a static flow restrictor. The energy intensity achieved in a device such as this is low; on the basis of the example in the publication, the working intensity which is exerted onto the free fatty acids is only less than 0.3 Wh/m³.

There are problems associated with the known technology. Thus, in the known processes for producing methyl esters of fatty acids, an acidic catalyst must be used, such as sulphuric acid, para toluene sulphonic acid, Lewis acids or solid ion-exchange resins, when it is desirable to keep both the temperature and the pressure of the esterification low. At the same time, the reaction times are relatively long. At high temperatures of over 250 °C and, correspondingly, at a high pressure, the reaction takes place without a catalyst, but high temperatures cause other unwanted reactions, such as dimerisation and polymerisation.

It is an aim of the present invention to eliminate at least some of the problems associated with the known technology and to generate a completely new industrially useful solution of producing biodiesel fuel.

According to the present invention, a reaction mixture is formed of raw material which comprises organic acids and of lower alcohol, which reaction mixture is brought into conditions in which the mixture cavitates, in order to esterify the organic acids. The esterification is continued for long enough to allow the acid number of the raw material, which comprises organic acids, to be reduced by at least 20 % from the original acid number. On the basis of the experiments, it seems that, under the described reaction conditions, it is possible to achieve a rapid esterification of the reactants, in which case even more than 50 % of the initial materials react and form esters in a very short reaction time.

More specifically, the method according to the present invention is mainly characterized by what is stated in the characterizing part of Claim 1.

Considerable advantages are achieved with the present invention. An example is that, according to the present invention, it is possible to produce high yields of an inexpensive biodiesel product (tall oil methyl ester = TOME), by esterification, from fatty acids of tall oil, especially from the crude fatty acid fraction. According to a preferred embodiment, when the esterification reactions are carried out rapidly by using a fast rotating power dispergator, which causes cavitation in the presence of methanol, which, in turn, generates rapid esterification of the reactants, the yield at this stage, calculated from the initial materials, is as high as 50-75 %. The reaction time is then even less than 0.1 second.

In the present invention, typically, fatty acids, resin acids or a mixture therof, are esterified. The raw material used is especially crude tall oil and more preferably an acid mixture generated from it, such as crude fatty acid mixture (CFA and, correspondingly, TOFA).

Esterification of the raw material is carried out for instance during the distillation of crude tall oil, and from the resin separation column, esters of tall oil resins are recovered as the bottoms product, which esters can be used as adhesives.

With the present invention, it is possible to conveniently adapt a standard tall oil distillery into a production plant for making fatty acid esters, and still retain the means to refine standard tall oil.

In the following, other benefits and details of the present invention are described with the help of a detailed explanation.

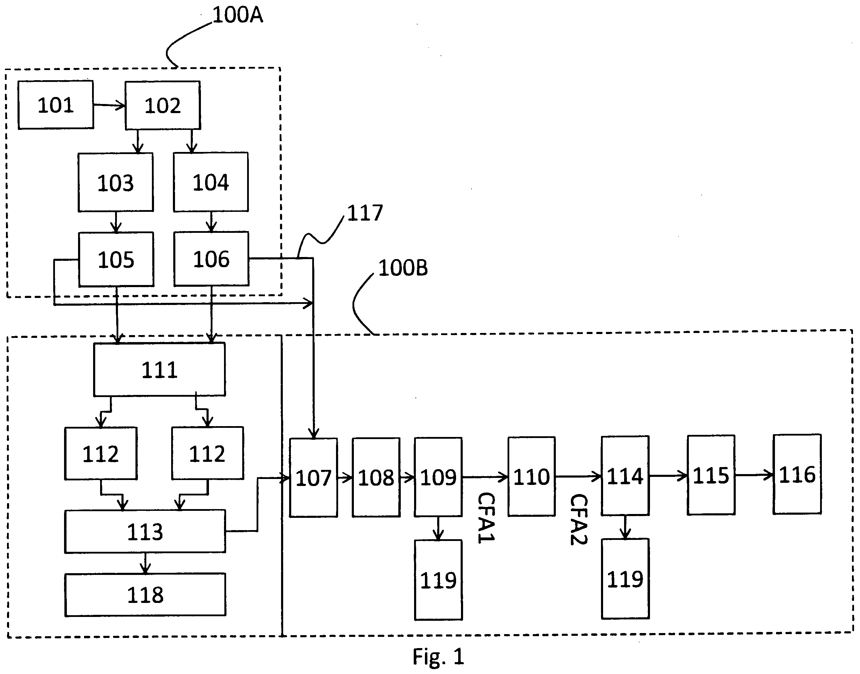

In the accompanying drawings, block diagrams show three alternative processes (figures 1-3), in which case the following reference numbers are used in the figures:

| **Reference number** | **Part of apparatus or stage of process** |
|---|---|
| | |
| 100A; 200A; 300A | Storage area |
| 101; 201; 301 | Formic acid |
| 102; 202; 302 | Protective esterification |
| 103; 203; 303 | CTO retention tank |
| 104; 204; 304 | CTO retention tank |
| 105; 205; 305 | CTO tank |
| 106; 206; 306 | CTO tank |
| 107; 207; 307 | Drying |
| 108; 208; 308 | Pitch separation (for instance OFH) |
| 109; 209; 309 | Resin separation column |
| 110; 210 | Primary oil separation |
| | |
| 310 | Methylation with homogeniser |
| 111; 211 | Methylation with homogeniser |
| 311 | Retention tank |
| 112; 212 | Retention tank |
| 312 | Methanol separation |
| 113; 213 | Methanol separation |
| 313 | Primary oil separation column |
| 114 | (TOFA) TOME distillation column |
| 214; 314 | TOME distillation column |
| 115; 215; 315 | Desulphurisation |
| 116; 216;316 | Biodiesel/Fatty acid store (TOME/TOFA) |
| | |
| 117; 217; 317 | Conventional fatty acid distillation |
| 118; 218; 318 | Methanol regeneration |
| 119;219; 319 | TOR methyl ester |

| | |
|---|---|
| CTO = Crude Tall Oil CFA = Crude Fatty Acid TOR = Tall Oil Resin TORME = TOR Methyl Ester | |

As described above, in the method according to the present invention, in order to convert vegetable-based organic acids, such as fatty acids or resin acids, into corresponding lower alcohol esters, a reaction mixture is formed of the organic acids and the lower alcohol, which mixture is brought into conditions where it cavitates, in order to esterify the organic acids and to lower the acid number of the raw material.

In our invention, we have made significant and unexpected discoveries during practical experiments, which discoveries totally change the processing of vegetable-based, especially wood-based initial material. Consequently, in our experiments we have discovered that it is possible to use efficiently a power dispergator to carry out the esterification reactions. Another important, and similarly surprising observation, is that, in such a stage of the esterification in which the fatty acids are partly or completely esterified, the fatty acid esters split into two different phases. This division takes place for instance in the storage tank.

In the present method, a raw material which comprises organic acids is processed, which raw material is typically sourced from wood either directly harvested or recovered via pulp cooking (for instance from wood oil or as pitch resin). The raw material used is for instance crude tall oil or an acid mixture generated from it. Preferably, the acids are concentrated in the raw material for instance by separating from the initial material other components which are unwanted in the esterification.

According to a preferred embodiment, the fatty acid esters are produced from a crude fatty acid mixture (CFA).

Typically, in Finland a common composition of crude fatty acid mixture is:

| | |
|---|---|
| Fatty acids | 90 % - acid number ∼188 mg KOH/g |
| Resin acids | 5.0 % |
| Neutral material | 5.0 % |

According to another embodiment, the raw material of esterification is a fatty acid mixture of tall oil (TOFA).

In a third embodiment, crude tall oil (CTO) is used which comprises, besides fatty acids, also resin acids and neutral material. However, preferably, the amount of resin acids is 20 % at the most, preferably 15 % of the total amount/weight of the acids in the raw material, and preferably, their quantity is at maximum 15 % of the total quantity of the raw material.

In the present method, fatty acids, resin acids or a mixture thereof are esterified with a lower alcohol, i.e. with a univalent C₁₋₄ alcanol, such as methanol or ethanol.

Methanol is more preferable.

Generally, stoichiometrically, at least as much univalent alcohol is used as there is esterifiable acid group in the organic acids, most suitably the molar amount of alcohol is approximately 1.1-20 fold compared with the acid equivalent.

According to a preferred embodiment, the reaction mixture comprises, besides organic acids and lower alcohol, also an acidic catalyst. Preferably, the catalyst used is a strong acid, and in the first reaction stage this is typically mineral acid or organic acid. An example of a suitable catalyst is sulphuric acid, the concentration of which is over 90 % and the quantity of which is approximately 0.1-10 %, especially approximately 0.5-5 %, most suitably approximately 1-3 %, of the weight of the acids to be esterified. Para toluene suphonic acid can also be used as catalyst, the amount being approximately 0.01-5 %, especially approximately 0.05-3 %, most suitably approximately 0.1-0.5 % of the weight of the acids to be esterified.

The reaction mixture formed of the intitial materials is brought into the first reaction stage, in which the acid number of the raw material is reduced in conditions of cavitation by at least 20 %, whereafter the reaction mixture is brought into the second reaction stage. In the second reaction stage, the reaction is continued for long enough to allow a significant part, at least 75 weight-% most suitably at least 85 weight-%, more preferably at least 90 weight-%, of the original organic acids to be esterified.

The second reaction stage is carried out under conditions in which the mixture does not cavitate, for instance in a retention tank. At this stage, the reaction mixture is left undisturbed or possibly it is mixed continuously or, most suitably, discontinuously. A strong acid is also used as the catalyst in the second esterification stage, for instance the same acid as in the first reaction stage, or an acidic ion-exchange resin. There is no need to remove the acid used in the first stage between the reaction stages, instead it is possible to bring the reaction mixture directly or unchanged into the second stage.

According to a preferred embodiment, the reaction between organic acids and alcohol is continued in the first reaction stage until the reaction mixture comprises at maximum 60 weight-%, preferably at maximum 50-25 weight-%, of the original organic acids. In the second stage, the esterification is continued for long enough to allow a significant part of the remaining acids to be esterified. Most suitably, at maximum 15 weight-% of the original organic acids are in acidic form.

Based on the above, a preferred embodiment of the present invention comprises a process in which the first esterification stage is carried out under cavitating conditions, and the second esterification stage is carried out under noncavitating conditions, in which case the acid number of the raw material (as regards the esterified acids) is reduced as a result of the overall process by at least 80 %, and even more (by 85-100 %).

According to a more preferred embodiment, the reaction mixture which is formed of the initial material and alcohol is subjected to conditions in which the mixture cavitates under large shear forces by leading it into a dispersion area. Most suitably, the dispersion area comprises at least one power dispergator, i.e. power mixer. Preferably, the dispersion area comprises one power dispergator or several power dispergators which are arranged in series, in which case the retention time of the mixture is shorter than approximately 1 second in each power dispergator.

In a preferred embodiment, one or several power mixers are used, each of which comprises at least one rotor. More preferably, one or several power mixers are used, which comprise rotors and stators.

The methylation is carried out at a temperature and pressure which, with regard to temperature, is equivalent to 90-99 % of the boiling point of the system, stated in degrees Celsius.

Most suitably, the reaction mixture is subjected to cavitation in the dispersion area, in which an overpressure is maintained, preferably approximately 1.1-20 bar absolute pressure, especially approximately 2-10 bar absolute pressure, especially approximately 2-5 bar absolute pressure, typically approximately 3-4 bar absolute pressure. Under the cavitation conditions in question, a temperature is maintained which - at the pressure in the cavitation area - is approximately 1-20 °C, especially approximately 2-10 °C lower than the boiling point of alcohol.

As described above, the method according to the present invention, has most suitably two stages, in which case, after the dispersion area, a retention area is arranged where the reaction mixture is left undisturbed, possibly under light stirring. In our practical experiments we have unexpectedly discovered that the esterification continued in the storage tank for instance during a period of 24 hours until it generated an acid number of -1- 0, when the initial esterification had been carried out with a power mixer. The product (TOME) which is esterified in the storage tank and methanol split into two different phases when the esterification for the crude fatty acid is carried out immediately after the resin column.

As mentioned above, according to a preferred embodiment, the reaction mixture which is generated in the cavitation stage is led as such (as a whole), i.e. without separation of its components, to a further reaction. For instance, a reaction carried out in a retention tank generates a mixture which is separated into two phases, from which mixture it is possible to separate the phases for instance by decanting.

In our experiments, the light phase comprised 82 % methanol, 0.5 % water and the remainder were fatty acids and fatty acid derivatives (esters). The heavy phase, in turn, comprised 44 % methanol, 2.5 % water and the remainder were fatty acids and fatty acid derivatives (esters) and a little resin acid. It is obvious that although such a phase-separation, which makes the processing somewhat difficult *per se,* is at the same time advantageous because less methanol distillation is required.

As shown above, the mixture, which is generated in the retention tank or which is otherwise a result of leaving the reaction mixture undisturbed, comprises a light stage, which mainly comprises methanol or a corresponding esterifying alcohol. It is possible to recover the alcohol and possibly reuse it after cleaning. According to a preferred embodiment, the lighter of the two phases which, after the esterification, are separated from the material, is as such reused for esterification.

Another preferred embodiment, which is part of the present invention, is a pretreatment of the raw material which is brought for esterification. This embodiment, in turn, is based on the unexpected observation that no phase-separation took place when the primary oil was removed by distillation from the crude fatty acids. When a phase-separation took place, the volume of the heavy phase was approximately 10 % of the total volume. When primary oils were distilled from the crude fatty acids, typically approximately 10 %, no phase-separation took place. In that case, fatty acids, mainly palmitinic acid, margaric acid and linolenic acid and, among others, neutral light alcohols of wood and a considerable part of the sulphur compounds in the feed, were removed from the crude fatty acids by distillation at the top of the column.

In another preferred pretreatment stage, which can be combined with the above mentioned pretreatment or which can be carried out independently, a raw material from which the esterified fatty acid product is produced, is protectively esterified by using formic acid before the separation of pitch and the separation of resin, in order to avoid losing fatty acids during the internal esterification generated by sterols and other alcohol-comprising groups. This solution is more closely described in our parallel PCT patent application WO 2008/139041.

The process described above is illustrated by the accompanying drawings. Three examples are presented, which differ slightly from each other and which are always chosen for instance according to the price of energy and the cost of capital.

The figures show the production of methyl ester of fatty acid/resin acid of tall oil during the crude tall oil process. The raw material which comprises organic acids is for instance sourced from wood either directly harvested or recovered via pulp cooking (the raw material is sourced for instance from wood oil or as pitch resin).

The reference numbers 100A, 200A and 300A each represents a storage area in which crude tall is stored and wherein, most suitably, the protective esterification processes 102, 202 and 302 of crude tall oil are carried out, and the reference numbers 100B, 200B and 300B each represents a distillation area, (i.e. in practice the distillation building), where the separation of primary oil 110, 210 and 313, and methylation 111, 211 and 310, conventional distillation of fatty acid, separation of methanol 113, 213 and 312, and desulphurisation 115, 215, 315, are carried out.

According to figure 1, for instance two lines (103 and 105; 104 and 106) have been arranged in the storage area, in which the crude tall oil is received from the pulp cooking and in which it is protectively esterified for instance in mixing tank 102 with formic acid, which acid comes from its own storage tank 101. With regard to the protective esterification, we refer to the solution which is described in our previous FI patent application 20070381. The protective esterification, which most suitably is carried out before the separation of pitch and resin, prevents the sterols from being esterified with the fatty acids within the mixture, which reduces the yield of fatty acids.

Preferably, the protective esterification is carried out with a power dispergator 102, which is used for treating the alcohol groups of acids of the initial material with formic acid.

The protective esterification is carried out by using the molar ratio between the initial materials, i.e. formic acid and organic acids, which ratio is approximately 1:1 - 2:1. The temperature of the esterification is approximately 10-140 °C, most suitably 50-70 °C. At higher temperatures, the esterification advances faster, but, at the same time, the colour of the product darkens.

The product is moved into the retention tank 103/104, the contents of which can be stirred. After that, the water and the excess acid are separated in the watering tanks 105/106.

The mixture which is generated from the protective esterification is led from the storage area, via drying 107 (i.e. dehydration) to the separation of pitch 108. In practice, the watered product is dried with an evaporator 107 and distilled in order to separate the pitch, for instance in a thin film evaporator 108.

In the next step, the resins are separated by distillation from the fatty acids 109, and the resins are removed from the base of the distillation column.

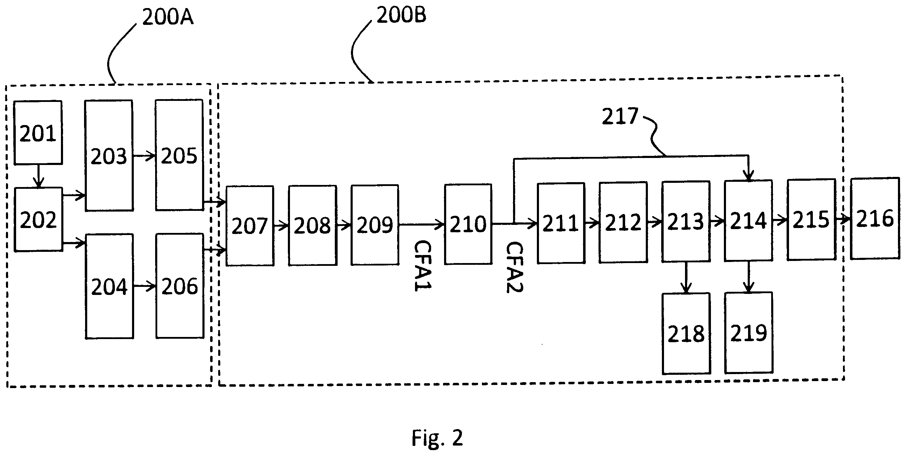

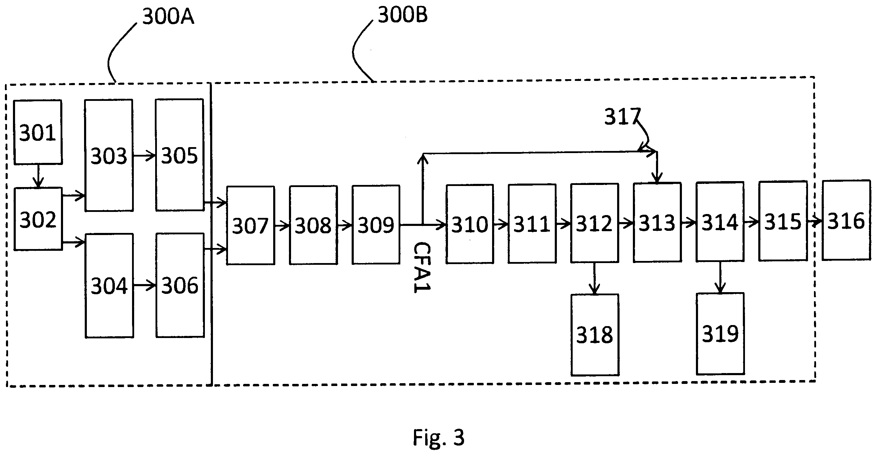

Figures 2 and 3 show similar processes in which the reference numbers at the beginning correspond to those in the explanation above.

The first crude fatty acid fraction which is generated is indicated in the drawings by the abbreviation CFA1 (Crude Fatty Acid 1).

In the solution according to figure 1, crude tall oil is methylated with a power dispergator, in which case part of the resin acids, too, are esterified, which makes their utilisation more effective.

In the solution according to figure 2, the primary oils are removed by distillation from the crude fatty acids, in which case, after the esterification, one phase is generated which comprises the esterified fatty acids, the unesterified fatty acids, the catalyst and the excess methanol, and the water which is released during the reaction.

Figure 3 shows an embodiment in which the crude fatty acids are directly esterified, without separation of primary oil, in which case two phases are generated.

As mentioned above, removal of primary oil is advantageous for further processing of the product, because phase-separation can be avoided by their removal. In the block diagram according to figure 1, the separation of primary oil is indicated by reference number 110 and in figure 2 it is indicated by number 210. After this separation, another, heavier crude fatty acid fraction, CFA2, is generated.

According to an embodiment, 0.1-20 weight-%, especially approximately 1-15 weight-%, most suitably approximately 5-10 weight-% of the raw material is removed by distillation in the primary oil column 110, before esterification. The distillation takes place at a pressure of approximately 5-50 mbar, especially approximately 10-30 mbar, and most suitably at a temperature of 180-280 °C, especially approximately 200-250 °C.

In our experiments, the upper phase comprised 74 % of the methanol fed, 11 % of the fatty acids and no resin acids at all, and 17 % of the entire amount of water generated. It is possible to reuse this phase for esterification, directly or after removal of the water, as shown in the figure (numbers 218, 318). The heavy phase, 10 % of the volume, comprised 84 % of all the fatty acids and 67 % of all the water.

In the processes described, the separation of methanol 113, 213, 312 is carried out for instance by distillation, after which regeneration of the methanol 118, 218, 318, i.e. dewatering, which can be carried out either by distillation or by drying with a molecular sieve. The actual product, i.e. TOME (TOFA methyl ester, i.e. methyl esters of fatty acids of tall oil), dries to become water-free during the same operation.

After that, in the following stage of the process, the separation of methyl esters of resin and the separation of resin is carried out using a thin film evaporator and/or distillation column 114, 214, 314. This is the column for distillation of TOME.

In our experiments, we discovered that after the methylation was carried out, in the presence of resin acids, also the resin acids were largely methylated (TOR methyl ester, i.e. methyl esters of resin acids of tall oil, figures 1, 2 and 3, reference numbers 119, 219 and 319). These methyl esters of resin acids of tall oil are well known to be good ingredients of sticker glues and more valuable than resin acids alone.

Figure 2 describes a situation where the primary oils are removed by distillation, before any other treatments. In the case of figure 1, they are distilled in a conventional way, after separation of resin. The procedure according to figure 2 is a more preferable embodiment, one which generates a better conversion into esters than the solution in figure 1. When acting according to the diagram of figure 3, two phases are generated, which are brought to further processing.

In our invention, we have quite unexpectedly obtained large conversions at retention times of less than 1 second, and the final conversion has taken place in the retention tank, during a period of approximately 24 hours. It seems that a reaction which is started with a power dispergator activates a post-reaction and there is no need to remove water.

In particular, when the catalyst used was sulphuric acid, it removed the water from the ester mixture. The percentage of the sulphuric acid used was 1 % of the weight of the methanol, and the molar ratio between methanol and fatty acids was 24.9.

In the power mixer, the sulphuric acid stage generated microscopic drops which absorb the water and shift the balance of the reaction in favour of the formation of esters.

The same happens when para toluene sulphonic acid is used, because it, too, is hydrophilic. Furthermore, because there is a sufficiently large amount of methanol, with water dissolved in it, its esterification-preventing concentration is significantly reduced.

The unexpected reaction rates can only be explained by the conditions, which are that the methanol is near enough to its boiling point that the mixture is continuously cavitating, in which case high local temperatures are generated, as a result of the cavitation bubbles collapsing.

It is obvious to experts that our invention works with other alcohols, too, such as ethanol, propanol and butanol, and corresponding C₁-C₄ alcanols. Naturally, their reaction times, conversions and suitable temperatures are different, and it is obvious that methanol is in practice the most cost-effective esterifying alcohol for producing biodiesel fuel.

### Example

In our experiments, the amount of crude fatty acid fed was 300 kg/h (acid number 188), of methanol 870 kg/h, the temperature after power dispersion was 98 °C and the counter pressure 250 kPa. Sulphuric acid (98 %) was added into methanol, 1 % of the weight of the methanol.

The power dispergator, which was used in our experiments, was of the commercial type Cavitron and it was run at a temperature of 98 °C and a pressure of 2.3 bars, in which the liquid clearly cavitated. The device comprised 3 mixing areas, a stator and a rotor, and the counter pressure was 2.3 bars, at a capacity of 5000 l/h. The steam pressure of pure methanol at a temperature of 98 °C is 2.8 bars, and thus, being diluted with water and fatty acid esters, it definitely boils, i.e. cavitates at the trailing edge of the rotor blade.

The engine power of the power dispergator was 22 kW and 75 % of its maximum speed of rotation, which was 2940 r/min, was used. The best esterification result was obtained at acid number 0.6 (1 sec with Cavitron and 7 days in the storage tank +20 °C). In that case, the molar ratio between methanol and fatty acid was 20. When the molar ratio was reduced from 20 → 10, the conversion of Cavitron decreased from 60 % → 50 %. After a stoppage time of 8 days, the final acid number was in all cases in the range of 5.3 → 0.6.

In other experiments, the catalyst used was para toluene sulphonic acid (PTSA), the amount of which was 0.4 % of the mass of the crude fatty acids.

With the present invention, it is possible to esterify crude tall oil (CTO), too. However, a series of tests in which crude tall oil (CTO), which comprised 29 % of resin, was directly methylated, gave a lower yield of esters than the direct esterification of crude fatty acids.

## Claims

1. A method of converting vegetable-based organic acids into corresponding lower alcohol esters, according to which method
- a raw material which comprises organic acids is, in order to esterify the acids, contacted with a lower alcohol in the presence of an acidic catalyst, said lower alcohol being a C₁-C₄ alcanol,
**characterized in that**
- a reaction mixture is formed of the raw material, which comprises organic acids, and the lower alcohol,
- the reaction mixture is brought into a first reaction stage in which it is subjected to conditions in which it cavitates in order to esterify the organic acids and to lower the acid number of the raw material by at least 20 %, after which the reaction mixture is brought into a second reaction stage in which the esterification reaction is continued for at least 6-200 hours.

2. The method according to Claim 1, **characterized in that** a mixture is formed of the raw material which comprises organic acids, of the lower alcohol and of the acidic catalyst, which mixture is led into a dispersion area in which it is subjected to conditions in which it cavitates under large shear forces.

3. A method according to Claim 1 or 2, **characterized in that** the reaction between the organic acids and the alcohol is continued under conditions of cavitation until the reaction mixture comprises at maximum 50 weight-%, of the original organic acids in acid form.

4. A method according to any of Claims 1-3, **characterized in that** the reaction mixture is fed into the dispersion area, which comprises at least one power dispergator, in order to bring the mixture into conditions in which it cavitates.

5. A method according to any of the preceding claims, **characterized in that** the reaction mixture is brought to cavitate in the dispersion area, in which an overpressure is maintained

6. A method according to any of the preceding claims, **characterized in that** under the cavitation conditions, a temperature is maintained which - at the pressure in the cavitation area - is 1-20 °C lower than the boiling point of alcohol.

7. The method according to any of the preceding claims, **characterized in that** the second reaction stage is carried out under conditions in which the mixture does not cavitate.

8. A method according to any of the preceding claims, **characterized in that** the reaction between the organic acids and the alcohol is continued in the first reaction stage until the reaction mixture comprises at maximum 60 weight-%, of the original organic acids, and in the second stage, the esterification is continued for long enough to allow a significant part of the remaining acids to be esterified, in which case most suitably at maximum 15 weight-% of the original organic acids are in acid form.

9. A method according to any of the preceding claims, **characterized in that** the raw material which comprises organic acids is sourced from wood either directly harvested or recovered via pulp cooking or an acid composition generated from these.

10. A method according to any of the preceding claims, **characterized in that** fatty acids, resin acids or a mixture thereof are esterified.

11. A method according to Claim 9 or 10, **characterized in that** the raw material used is crude tall oil, crude fatty acid mixture or crude resin acid.

12. A method according to any of the preceding claims, **characterized in that**, after the esterification, the lighter phase of the two materials which are separated into two phases is reused as such for esterification.

13. A method according to any of the preceding claims, **characterized in that** the raw material, from which the esterifiable fatty acid product is produced, is protectively esterified with formic acid, before the separation of pitch and the separation of resin, in order to avoid losing the fatty acids in the internal esterification with sterols and other groups which comprise alcohols.

14. A method according to any of the preceding claims, **characterized in that** the raw material used is crude tall oil or a crude fatty acid mixture generated from it, in which case the esterification is carried out in association with the distillation of crude tall oil, and from the resin separation column, esters of tall oil resins are recovered as the bottoms product, which esters can be used as adhesives.

15. A method according to any of the preceding claims, **characterized in that** the catalyst used is a strong acid, which is a mineral acid or an organic acid or an acidic ion-exchange resin.

16. A method according to any of the Claims 15, in which method a homogenous catalyst is used, **characterized in that** the catalyst is added into the methanol.

17. A method according to any of the preceding claims, **characterized in that** the alcohol used is methanol, in order to produce the corresponding methyl esters.

18. A method according to any of the preceding claims, **characterized in that** the initial material, which comprises acids, is methylated by using a power mixer, which comprises rotors and stators.

## Patentansprüche

1. Verfahren zur Umwandlung von organischen Säuren auf pflanzlicher Basis in entsprechende niedere Alkoholester, wobei gemäß dem Verfahren
- ein Rohstoff, der organische Säuren umfasst, um die Säuren zu verestern, mit einem niederen Alkohol in Gegenwart eines sauren Katalysators in Kontakt gebracht wird, wobei der niedere Alkohol ein C₁-C₄-Alkanol ist,
**dadurch gekennzeichnet, dass**
- aus dem Rohstoff, der organische Säuren umfasst, und dem niederen Alkohol ein Reaktionsgemisch gebildet wird,
- das Reaktionsgemisch in eine erste Reaktionsstufe gebracht wird, in der es Bedingungen ausgesetzt wird, unter denen es kavitiert, um die organischen Säuren zu verestern und die Säurezahl des Rohstoffs um mindestens 20 % zu senken, wonach das Reaktionsgemisch in eine zweite Reaktionsstufe gebracht wird, in der die Veresterungsreaktion für mindestens 6-200 Stunden fortgesetzt wird.

2. Verfahren nach Anspruch 1, **dadurch gekennzeichnet, dass** ein Gemisch aus dem Rohstoff, der organische Säuren umfasst, dem niederen Alkohol und dem sauren Katalysator gebildet wird, das in einen Dispersionsbereich geleitet wird, in dem es Bedingungen ausgesetzt wird, in denen es unter großen Scherkräften kavitiert.

3. Verfahren nach Anspruch 1 oder 2, **dadurch gekennzeichnet, dass** die Reaktion zwischen den organischen Säuren und dem Alkohol unter Kavitationsbedingungen fortgesetzt wird, bis das Reaktionsgemisch maximal 50 Gew.-% der ursprünglichen organischen Säuren in Säureform umfasst.

4. Verfahren nach einem der Ansprüche 1-3, **dadurch gekennzeichnet, dass** das Reaktionsgemisch in den Dispersionsbereich, der mindestens einen Energiedispergator umfasst, eingebracht wird, um das Gemisch in Bedingungen zu bringen, in denen es kavitiert.

5. Verfahren nach einem der vorstehenden Ansprüche, **dadurch gekennzeichnet, dass** das Reaktionsgemisch im Dispersionsbereich zur Kavitation gebracht wird, wobei ein Überdruck aufrechterhalten wird.

6. Verfahren nach einem der vorstehenden Ansprüche, **dadurch gekennzeichnet, dass** unter den Kavitationsbedingungen eine Temperatur aufrechterhalten wird, die - bei dem Druck im Kavitationsbereich - 1-20°C niedriger als der Siedepunkt von Alkohol ist.

7. Verfahren nach einem der vorstehenden Ansprüche, **dadurch gekennzeichnet, dass** die zweite Reaktionsstufe unter Bedingungen durchgeführt wird, bei denen das Gemisch nicht kavitiert.

8. Verfahren nach einem der vorstehenden Ansprüche, **dadurch gekennzeichnet, dass** die Reaktion zwischen den organischen Säuren und dem Alkohol in der ersten Reaktionsstufe fortgesetzt wird, bis das Reaktionsgemisch maximal 60 Gew.-% der ursprünglichen organischen Säuren umfasst, und in der zweiten Stufe die Veresterung so lange fortgesetzt wird, um Veresterung eines wesentlichen Teils der verbleibenden Säuren zu erlauben, wobei in diesem Fall höchstens 15 Gew.-% der ursprünglichen organischen Säuren in Säureform vorliegen.

9. Verfahren nach einem der vorstehenden Ansprüche, **dadurch gekennzeichnet, dass** der Rohstoff, der organische Säuren umfasst, aus Holz gewonnen wird, das entweder direkt geerntet oder durch Kochen von Zellstoff oder einer daraus erzeugten sauren Zusammensetzung gewonnen wird.

10. Verfahren nach einem der vorstehenden Ansprüche, **dadurch gekennzeichnet, dass** Fettsäuren, Harzsäuren oder ein Gemisch davon verestert werden.

11. Verfahren nach Anspruch 9 oder 10, **dadurch gekennzeichnet, dass** der verwendete Rohstoff Rohtallöl, Rohfettsäuregemisch oder Rohharzsäure ist.

12. Verfahren nach einem der vorstehenden Ansprüche, **dadurch gekennzeichnet, dass** nach der Veresterung die leichtere Phase der zwei Materialien, die in zwei Phasen getrennt sind, als solche zur Veresterung wiederverwendet wird.

13. Verfahren nach einem der vorstehenden Ansprüche, **dadurch gekennzeichnet, dass** der Rohstoff, aus dem das veresterbare Fettsäureprodukt hergestellt wird, vor der Trennung von Pech und der Trennung von Harz schützend mit Ameisensäure verestert wird, um den Verlust der Fettsäuren bei der inneren Veresterung mit Sterolen und anderen alkoholhaltigen Gruppen zu vermeiden.

14. Verfahren nach einem der vorstehenden Ansprüche, **dadurch gekennzeichnet, dass** der verwendete Rohstoff Rohtallöl oder ein daraus erzeugtes Rohfettsäuregemisch ist, wobei in diesem Fall die Veresterung in Verbindung mit der Destillation von Rohtallöl durchgeführt wird, und aus der Harztrennsäule Ester von Tallölharzen als Bodenprodukt gewonnen werden, wobei die Ester als Klebstoffe verwendet werden können.

15. Verfahren nach einem der vorstehenden Ansprüche, **dadurch gekennzeichnet, dass** der verwendete Katalysator eine starke Säure ist, die eine Mineralsäure oder eine organische Säure oder ein saures lonenaustauscherharz ist.

16. Verfahren nach einem der Ansprüche 15, wobei ein homogener Katalysator verwendet wird, **dadurch gekennzeichnet, dass** der Katalysator dem Methanol zugesetzt wird.

17. Verfahren nach einem der vorstehenden Ansprüche, **dadurch gekennzeichnet, dass** der verwendete Alkohol Methanol ist, um die entsprechenden Methylester herzustellen.

18. Verfahren nach einem der vorstehenden Ansprüche, **dadurch gekennzeichnet, dass** der Ausgangsstoff, der die Säuren umfasst, unter Verwendung eines Kraftmischers, der Rotoren und Statoren umfasst, methyliert wird.

## Revendications

1. Procédé de conversion d'acides organiques à base végétale en esters d'alcool inférieur correspondants, selon ce procédé
- une matière première comprenant des acides organiques est, pour estérifier les acides, mise en contact avec un alcool inférieur en présence d'un catalyseur acide, ledit alcool inférieur étant un alcanol en C₁-C₄,
**caractérisé en ce que**
- un mélange réactionnel de la matière première est formé, qui comprend des acides organiques et de l'alcool inférieur,
- le mélange réactionnel est amené dans une première étape réactionnelle dans laquelle il est soumis à des conditions dans lesquelles il créé une cavitation pour estérifier les acides organiques et abaisser l'indice d'acide de la matière première d'au moins 20%, après quoi le mélange réactionnel est amené dans une seconde étape réactionnelle dans laquelle la réaction d'estérification est poursuivie pendant au moins 6 à 200 heures.

2. Procédé selon la revendication 1, **caractérisé en ce qu'**un mélange de la matière première est formé comprenant des acides organiques, de l'alcool inférieur et du catalyseur acide, ledit mélange est conduit dans une zone de dispersion dans laquelle il est soumis à des conditions dans lesquels il créé une cavitation soumis à d'importantes forces de cisaillement.

3. Procédé selon la revendication 1 ou 2, **caractérisé en ce que** la réaction entre les acides organiques et l'alcool est poursuivie dans des conditions de cavitation jusqu'à ce que le mélange réactionnel comprenne au maximum 50% en poids des acides organiques d'origine sous forme acide.

4. Procédé selon l'une quelconque des revendications 1 à 3, **caractérisé en ce que** le mélange réactionnel est introduit dans la zone de dispersion, qui comprend au moins un disperseur de puissance, pour amener le mélange dans des conditions dans lesquelles il créé une cavitation.

5. Procédé selon l'une quelconque des revendications précédentes, **caractérisé en ce que** le mélange réactionnel est amené à créer une cavitation dans la zone de dispersion dans laquelle une surpression est maintenue.

6. Procédé selon l'une quelconque des revendications précédentes, **caractérisé en ce que** dans les conditions de cavitation, on maintient une température qui - à la pression dans la zone de cavitation - est inférieure de 1 à 20°C au point d'ébullition de l'alcool.

7. Procédé selon l'une quelconque des revendications précédentes, **caractérisé en ce que** la seconde étape réactionnelle est réalisée dans des conditions dans lesquelles le mélange ne créé pas de cavitation.

8. Procédé selon l'une quelconque des revendications précédentes, **caractérisé en ce que** la réaction entre les acides organiques et l'alcool est poursuivie dans la première étape réactionnelle jusqu'à ce que le mélange réactionnel comprenne au maximum 60% en poids des acides organiques d'origine et dans la seconde étape, l'estérification est poursuivie pendant suffisamment longtemps pour permettre d'estérifier une partie importante des acides restants, auquel cas, de la manière la plus appropriée, au maximum 15% en poids des acides organiques d'origine sont sous forme acide.

9. Procédé selon l'une quelconque des revendications précédentes, **caractérisé en ce que** la matière première qui comprend des acides organiques provient du bois soit directement récolté, soit récupéré via une cuisson de pâte ou une composition acide produite à partir de ceux-ci.

10. Procédé selon l'une quelconque des revendications précédentes, **caractérisé en ce que** des acides gras, des acides résiniques ou un mélange de ceux-ci sont estérifiés.

11. Procédé selon la revendication 9 ou 10, **caractérisé en ce que** la matière première utilisée est le tallol brut, un mélange d'acides gras bruts ou d'acides résiniques bruts.

12. Procédé selon l'une quelconque des revendications précédentes, **caractérisé en ce que**, après l'estérification, la phase plus légère des deux matériaux qui sont séparés en deux phases est réutilisée telle quelle pour l'estérification.

13. Procédé selon l'une quelconque des revendications précédentes, **caractérisé en ce que** la matière première à partir de laquelle le produit d'acides gras estérifiable est produit est estérifiée de manière protégée avec de l'acide formique avant la séparation du brai et la séparation de la résine, de manière à éviter de perdre les acides gras dans l'estérification interne avec des stérols et d'autres groupes qui comprennent des alcools.

14. Procédé selon l'une quelconque des revendications précédentes, **caractérisé en ce que** la matière première utilisée est le tallol brut ou un mélange d'acides gras brut généré à partir de celui-ci, auquel cas l'estérification est effectuée en association avec la distillation du tallol brut, et à partir de la colonne de séparation de résine, des esters de résines de tallol sont récupérés en tant que produit de queue, ces esters pouvant être utilisés comme adhésifs.

15. Procédé selon l'une quelconque des revendications précédentes, **caractérisé en ce que** le catalyseur utilisé est un acide fort, qui est un acide minéral ou un acide organique ou une résine échangeuse d'ions acide.

16. Procédé selon l'une quelconque des revendications 15, dans lequel on utilise un catalyseur homogène, **caractérisé en ce que** le catalyseur est ajouté dans le méthanol.

17. Procédé selon l'une quelconque des revendications précédentes, **caractérisé en ce que** l'alcool utilisé est le méthanol pour produire les esters méthyliques correspondants.

18. Procédé selon l'une quelconque des revendications précédentes, **caractérisé en ce que** le matériau initial, qui comprend des acides, est méthylé en utilisant un mélangeur à puissance, qui comprend des rotors et des stators.
